# EUROPEAN PATENT SPECIFICATION

(11) **EP 0 673 627 B1**
(45) Date of publication and mention of the grant of the patent: **05.01.2000**
(21) Application number: 95301906.4
(22) Date of filing: 22.03.1995
(51) Int. Cl.: A61B 17/36, A61N 5/06

(54) **Catheter with optical fiber**
Katheter mit Lichtleitfaser
Cathéter à fibre optique

(30) Priority: 23.03.1994 JP 5195094
(43) Date of publication of application: 27.09.1995
(73) Proprietor: Hashimoto, Yasuo, Yokohama-shi, Kanagawa-ken 247 (JP); Hamamatsu Photonics K.K., Shizuoka-ken 435-8558 (JP); Yamaguchi, Noboru, Yokohama-shi, Kanagawa-ken 235 (JP)
(72) Inventor: Hashimoto, Yasuo, Yokohama-shi, Kanagawa-ken, 247 (JP); Hirano, Toru, c/o Hamamatsu Photonics K.K., Hamamatsu-shi,Shizuoka-ken, 435 (JP); Yamaguchi, Noboru, Yokohama-shi, Kanagawa-ken, 235 (JP)
(74) Representative: Overbury, Richard Douglas

(56) References cited:
- EP-A- 0 195 375
- EP-A- 0 394 446
- EP-A- 0 441 040
- EP-A- 2 122 253
- WO-A-94/00052
- US-A- 4 740 047
- US-A- 4 973 848
- US-A- 5 151 096

## Description

### BACKGROUND OF THE INVENTION

### Field of the Invention

The present invention relates to a cancer therapeutic instrument for use in the treatment of cancers and neoplasm, and more particularly a cancer therapeutic instrument for use in the treatment of the cancers originated, for example, in areas which involve a great deal of difficulty for performing resection operations such as organs and the like, pancreatic carcinoma, lung cancer, brain tumor, cancers originating in the brain stem area. The invention also applies to cancers which it is not desirable to be resected from the point of view of beauty and keeping the function, for example, breast cancer, head and neck cancer, tongue cancer and uterine cancer.

### Description of the Related Art

Currently, cancer is one of the most terrible diseases which are left to mankind, and elucidation of its fundamental cause and the therapy are in a developing state.

As typical therapy, which is considered at present to be effective, surgical resection, radiation therapy and chemotherapy are well known and the most frequently employed.

Among those therapeutic methods, surgical resection is deemed to be most effective and is most frequently employed. On the other hand, for malignant tumors originating deep inside the body for which surgical resection cannot be employed, radiation therapy and chemotherapy are frequently used in combination. However, it often happens that both radiation therapy and chemotherapy cause strong adverse reactions, and radiation therapy and chemotherapy have remarkably lower complete cure rates compared with surgical resection and involve the danger of relapses.

Recently, in view of the foregoing, there has been vigorous study of cancer therapy by physical treatments which involve no adverse reaction. As typical treatments, hyperthermia and phototherapy using a photosensitizer are known.

In the case of hyperthermia a cancer cell is extinguished by heating and/or cooling thereof. It has been reported that merely keeping a cancer cell at a temperature, for example 5°C higher than body temperature (which would cause relatively less damage to peripheral normal cells) throughout a definite time, may bring a great treatment effect.

On the other hand, phototherapy is based on the fact that irradiation with a specified wavelength of activation light of a cell which has absorbed a photosensitizer leads to the absorption of optical energy by the photosensitizer so that activating oxygen, which will destroy the cell, emanates. This treatment utilizes the fact that the excretion rate of the photosensitizer after injection is extremely low in a cancer cell compared with that of a normal cell. Specifically, the excretion rate of a cancer cell is less than 1/3 (about 1/5) of a normal cell.

The present invention is involved in the phototherapy, and thus the phototherapy will be described in detail hereinafter.

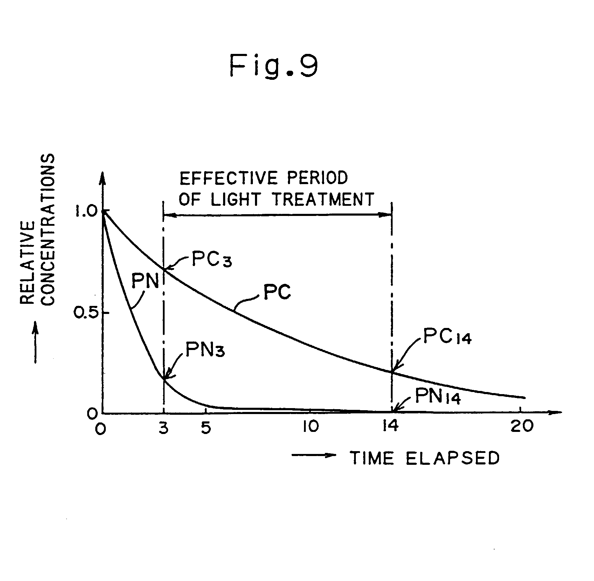

Fig. 9 is a graph showing the relation between time elapsed and the relative concentrations of ATX-S10 in cells. The time point when an ATX-S10, which is a photosensitizer, is injected into the body is selected as a starting point and the relative concentrations of the ATX-S10 remaining in cells at various times is shown. The graph PN denotes the residual concentrations of the ATX-S10 within the normal cells; and the graph PC the residual concentrations of the ATX-S10 within the cancer cells.

Once a certain standby time has elapsed (about 3 hours) after plenty of photosensitizer is injected into the affected part and its peripheral cells by direct injection, intravenous injection and the like, the normal cells have discharged almost all photosensitizer, whereas the photosensitiser stays in the cancer cells; this state is known as the light treatment feasible state. In such a light treatment feasible state, irradiation with a specified wavelength of activation light leads to the emission of activating oxygen which will destroy the cancer cells in which almost all photosensitizer stays. Since almost no activating oxygen emanates from within the normal cells which have discharged almost all photosensitizer, the cancer cells are selectively extinguished.

The duration for effective light treatment i.e the duration of the light treatment feasible state following injection of the photosensitizer is from 3 hours after injection of the photosensitizer and terminates after 14 hours. After this time the cancer cells have discharged almost all photosensitizer. Incidentally, Fig. 9 shows an ATX-S10 by way of example. The standby time after injection of the photosensitizer and the duration of effective light treatment will vary by about one order of magnitude in accordance with a sort of the photosensitizer.

The gist of this treatment resides in the fact that activation light is continuously projected onto the affected part as uniformly as possible during the term of the effective for light treatment. The amount of irradiation light is controlled in such a manner that cell-inside-concentrations of activating oxygen which causes cells to be extinguished is restricted to a very low value. Hence the death rate of normal cells is within the permitted limit for normal cells. In contrast, the cell-inside-concentrations of activating oxygen assumes a value such that death rate of the cancer cells is near to 100%.

Previously, to project the activation light onto an affected part, an optical fiber was directly inserted into the affected part and activation light is transmitted through the optical fiber. However, according to this scheme:
(a) The optical fiber cannot be moved during the treatment and the irradiation area or the therapeutic area is extremely limited;
(b) To project light from the tip of the optical fiber in all directions, it is necessary to cut the tip of the optical fiber, for example, into a cone shape. Processing of an optical fiber in this way is difficult and then the optical fiber is very expensive;
(c) To perform the treatment repeatedly, it is necessary to insert the optical fiber whenever the treatment is performed. It is a great burden to both a patient and a doctor;
(d) Since the therapeutic area is narrow, it is difficult to cure completely large cancer and tumor; and
(e) There are needs of sterilization of the optical fiber before use and re-sterilization of the optical fiber after each use or solid waste disposal. Usually, it is needed to provide an optical fiber having a length not less than 1m, and the optical fiber is of a compound material. Consequently, the sterilization and the solid waste disposal are troublesome and thus the optical fiber is poor in operational efficiency.

EPA 0 195 375 discloses a catheter for laser anginosurgery. Optical fibres are mounted in a catheter for insertion into an artery and provide a controlled delivery of laser light. The ends of the optical fibres are adjusted as required for a given application and fixed in place near the distal end of the catheter so that the output can be controlled. A protective optical shield mechanically displaces blood.

US 5151096 discloses a laser catheter diffuser for photodynamic treatment of cancer tumours. The apparatus includes an unclad fibre-optic core having a diffuser matrix coating and a transparent tubular sleeve. The laser catheter diffuser is formed by inserting the unclad fibre-optic core into the sleeve and applying the diffuser matrix which hardens by standard curing to seal the laser catheter diffuser.

US 4740047 discloses an optical fibre for lateral beaming of a laser beam to treat internal parts of the body. Light is reflected laterally from the inclined end of the optical fibre by means of a reflective coating. The distal end of the optical fibre and the tubular member which surrounds the optical fibre are attached to one another in an air-tight manner by epoxy adhesive.

US 4973848 discloses an apparatus in which two laser beams concurrently provide analysis and treatment of a desired region. Automatic adjustment of the treatment laser beam is effected depending on the results measured by the analysis laser beam. The beams are provided by optical fibres which protrude from the end of a conventional endoscope.

WO 94/00052 discloses an ultrasonic imaging probe for providing two- or three-dimensional representations of scanned tissue. The ultrasound probe is positioned accurately by means of a complex position translator and "data-slices" which are taken at pre-determined intervals are processed to produce an ultrasound image of the desired region

### SUMMARY OF THE INVENTION

In view of the foregoing, it is therefore an object of the present invention to provide a cancer therapeutic instrument capable of providing effective light treatment by irradiating the affected part with activation light over a wide range. The cancer therapeutic instrument is excellent in operational efficiency, inexpensive and avoids the disadvantages of prior art instruments.

According to the present invention, there is provided a cancer therapeutic instrument comprising: a catheter having a tube whose tip is closed at the distal end and which is adapted to be inserted into a subject to be treated, an optical fibre adapted to be slidably disposed inside the tube, a light source emitting activation light which is arranged so as to introduce the activation light into one end of the optical fibre contained within the tube so that the activation light travels within the optical fibre and emanates from the other end of the optical fibre, and a driving mechanism, characterised in that the driving mechanism comprises a motor, a fixed bearing and a reel, wherein the motor and the reel are joined via a rod incorporating a screw thread and being associated with a fixed bearing, and wherein the optical fibre is secured to the reel by means of reel fixture so that operation of the motor serves to displace the reel relative to the fixed bearing and the tube so as to drive that part of the optical fibre between the tip and reel fixture in such a manner that the optical fibre within tube is capable of periodic reciprocation in the longitudinal direction of the tube and periodic rotational motion about the longitudinal axis of the tube.

The terminology "tube whose tip is closed" implies that it is acceptable either that the tip of the tube itself is closed, or that the tip of the tube is closed by a stopper or the like mounted in the opening of the tip.

In an embodiment of the present invention, it is preferable that the optical fibre has at its tip an oblique plane with respect to the longitudinal direction of said optical fibre.

In an embodiment of the present invention, the tube is provided with a scatter member adapted to scatter predetermined activation light, and the optical fibre whose tip is disposed inside the tube emits from its tip activation light towards the scatter member.

It is preferable that the scatter member is made of plastics material containing polyurethane or polyacetal.

In an embodiment of the present invention, the emission solid angle (which indicates the spread of light emitted from the optical fiber and which is defined as an area in which luminous density of emission light on a spherical surface produced by the tip centre of the optical fiber) in the center becomes not less than 0.37 times a maximum, and the tip of the optical fiber is formed as a plane having an emission solid angle of not less than 0.12 steradian.

In addition, it is preferable that the tube is formed to provide a cutting plane whose area decreases in the plane perpendicular to the longitudinal direction of the tube as it approaches the tip of the tube which is to be inserted into the subject to be treated.

It is preferable that the light source is arranged to continuously or stepwise increase the amount of activation light entering the optical fiber as incident light with the elapse of time.

Because the optical fiber is disposed slidably inside the tube, a patient does not feel pain even if the optical fiber is subjected to reciprocation or rotational motion. Further, insertion and drawing operations of the optical fiber involve no danger that a patient is damaged in every operation. Hence, it is facilitated that treatment can be performed on several occasions on a divisional basis. Incidentally, it is permitted to use tubes with a diameter of the order of 0.5mm - 1.5mm, and there is little side effect such as the bleeding or the like due to the insertion of the tube into the affected part.

It is sufficient that strict sterilization of the optical fiber before use or treatment is performed for only the tube. Further, after the treatment it is possible to easily perform solid waste disposal for the tube, which is much inexpensive than the optical fiber, for example by burning the tube. In addition, the cancer therapeutic instrument is excellent in operational efficiency.

Further, reciprocating the optical fiber makes it possible to obtain a broad irradiation area in the longitudinal direction of the tube, thereby coping with the large affected part.

To cut obliquely the tip of the optical fiber is implemented inexpensively, and the combined use of rotational motion and the oblique cut of the optical fiber makes it possible to obtain a broad irradiation area around the tube.

Periodical reciprocation and/or rotational motion of the optical fiber make it possible to continuously irradiate with activation light for a long time throughout a broad light irradiation area. In addition it is possible to prevent fusion and carbonization of the thin tube due to heat of light irradiation, thereby enabling the use of the tube for a long time since no particular portion of the tube is continuously irradiated with light.

When a scatter member is provided inside a tube such an instrument is suitable for treatment of small affected parts. The use of a scatter member makes it possible, even with out reciprocation and/or rotational motion of the optical fiber to form the tip of the optical fiber as a light source which may emit light beams in the all directions. It is acceptable either that the scatter member is adapted to serve as a stopper for the tip of the tube as well; that the tip of the tube is originally made in such a configuration that the scatter member is disposed; that the scatter member is inserted from the tip having an opening and thereafter the opening is closed; or that the scatter member is inserted from the rear end of the tube having an opening into the tube. In the case where the scatter member is provided, there is no need to cut obliquely the tip of the optical fiber, and it is acceptable that the optical fiber has at the tip thereof a plane having a cut perpendicular to the longitudinal direction of the optical fiber, and the scatter member has at a position coming face to face with the tip of said optical fiber, a plane expanding in parallel with said plane of the optical fiber.

According to one embodiment the tip of an optical fiber, which is disposed inside a tube, is equipped with a scatter plane so that activation light is emitted from the tip of the optical fiber with a spread not less than a predetermined solid angle.

In this embodiment the emission solid angle (which indicates the spread of light emitted from the optical fiber and which is defined as an area in which luminous density of emission light on a spherical surface produced by the tip center of the optical fiber) becomes not less than 0.37 times a maximum in the centre. The scatter plane of the tip of the optical fiber (which is defined as a plane with the emission solid angle) is not less than 0.12 steradian. The reason that these values are chosen is that such a degree of scatter plane can be obtained simply and inexpensively through rough grinding by a file and the like, and also because a broad area of the affected part is irradiated with activation light. Effective treatment can be obtained, through a spread of activation light to the extent that the above definition is satisfied even if an independent scatter member or the like is not provided in addition to the optical fiber. When the optical fiber is slidably disposed inside the tube and is reciprocated, it is possible to obtain a broader light irradiation area, thereby coping with large affected parts. Further, providing periodic reciprocation permits a broad light irradiation area to be uniformly irradiated with activation light.

In any of the embodiments of the present invention as described above, if polyurethane or polyacetal is selected as the main raw materials, it is possible to provide a tube having the necessary light transmission ability for therapeutic instruments, and having a soft structure which means that there is no danger that the human body is damaged, and the tube is not toxic to the human body. Further, in case of polyacetal, there is the advantage that it is hard to be carbonized even if the tube is irradiated with strong light.

Further, sharpening the tip of the tube facilitates the tube to be inserted into the affected part.

As shown in Fig. 9, even in effective period of light treatment, concentrations of photosensitizer within the normal cells continue to be decreased, and thus activating oxygen is hardly produced within the normal cells even with irradiation of strong activation light. Consequently, it is possible to obtain the further effect of treatment by irradiation with stronger activation light with the elapse of time.

Thus, by using the cancer therapeutic instrument of the present invention as described above, it is possible to provide inexpensively tubes and optical fibers capable of withstanding the use for long time and also obtaining a broad light irradiation area. Further, it is excellent in operational efficiency and in sanitary management since the tube, which is inexpensive and is feasibly disposed by burning, may be used on a throw-away basis for each patient or each treatment.

### BRIEF DESCRIPTION OF THE DRAWINGS

Fig. 1 is a typical illustration of a cancer therapeutic instrument according to an embodiment of the present invention;
Fig. 2 is a partially enlarged view showing the tip portion of a tube inserted into the affected part and the tip portion of an optical fiber inserted into the tube;
Figs. 3(A)-3(C) are each a view useful for understanding a variation of irradiation area of laser beams according to movement of the optical fiber;
Fig. 4 is a view showing a state in which two tubes are inserted into the affected part, and in addition a heat pipe is inserted into the affected part;
Fig. 5 is a partially enlarged view showing the tip portion of a tube and the tip portion of an optical fiber. Although Fig.5 does not relate to an embodiment of the invention as defined in the claims, it appears to be useful for its understanding.
Fig. 6 is a view showing the tip portion of an optical fiber and the state of spread of emitted light according to still another embodiment of a cancer therapeutic instrument of the present invention;
Figs. 7 (A) and (B) are each a view showing the tip of a tube;
Fig. 8 is a graph which exemplarily shows the variation of the amount of light emitted from a laser light source according to time elapsed; and
Fig. 9 is a graph showing the relation between the time elapsed, starting from the time when photosensitizer is injected into the body, and the relative concentrations of the photosensitizer remaining in cells.

### DESCRIPTION OF THE PREFERRED EMBODIMENTS

Hereinafter, there will be described embodiments of the present invention.

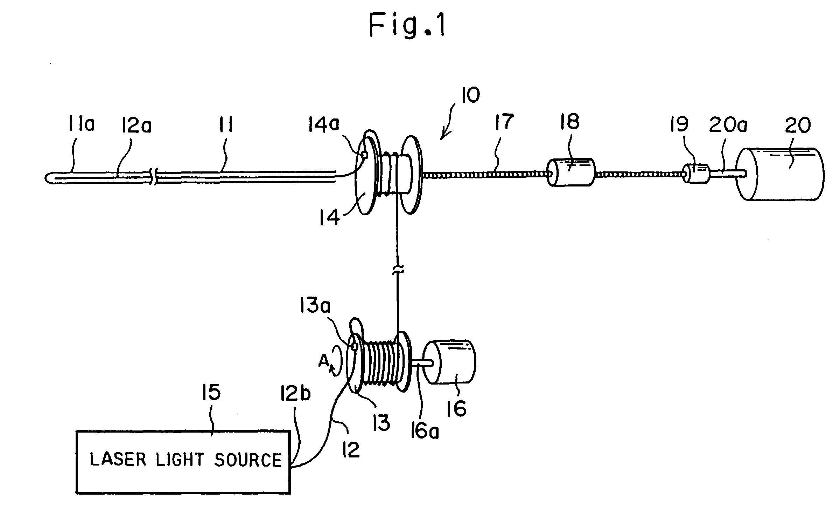

Fig. 1 is a typical illustration of a cancer therapeutic instrument according to an embodiment of the present invention.

In Fig. 1, the cancer therapeutic instrument 10 is equipped with a throw-away type of tube 11 the tip portion 11a of which is inserted into the affected part involved in the cancer or the tumor within the body of the patient. The tube 11 is made of, for example, polyacetal and is of a diameter of the order of about 1mm.

The cancer therapeutic instrument 10 is further equipped with an optical fiber 12 which is wound into a first reel 13 and a second reel 14 and is fixed on the first reel 13 and the second reel 14 through fixtures 13a and 14a, respectively. The tip portion 12a of the optical fiber 12 at the end of the second reel 14 is inserted into the tube 11 and reaches the tip portion 11a. An incident end 12b of the optical fiber 12 is connected to a laser light source 15. The laser light source 15 emits a beam of laser light having a wavelength utilized as the activation light. The emitted beam of laser light is introduced to the optical fiber 12 through the incident end 12b of the optical fiber 12. The laser light source 15 can emit laser light which is variable in a quantity of light. The quantity of light is varied with the elapse of time by a control circuit (not illustrated). Details will be described later. The laser beam introduced to the optical fiber 12 travels through the optical fiber 12 and emanates from the tip inserted into the tube 11.

The first reel 13 is coupled with a shaft 16a of a torque motor 16 so as to be actuated in an arrow direction A shown in the figure. Fixed on the second reel 14 is a rod 17 over the surface of which male screws are formed. The rod 17 is engaged with a fixed bearing 18 on a spiral basis, and is coupled through a coupling member 19 to a shaft 20a of a reversible motor 20. The fixed bearing 18 is fixed on a base or the like (not illustrated). The reversible motor 20 is rotatable both forward and backward. Providing a guide member (not illustrated) prevents the motor main body from being rotated, and permits the motor to overall move forward and backward (left and right in the figure).

When the reversible motor 20 rotates forward, the rod 17 also rotates forward. Thus, since the rod 17 is engaged with the fixed bearing 18 on a spiral basis, the rod 17 moves forward together with the reversible motor 20, so that the second reel 14 is rotated and advanced. At that time, the second reel 14 pulls the optical fiber 12 wound into the first reel 13 so as to be wound into the second reel 14. Whereas the tip portion 12a of the optical fiber 12 is squeezed into the tube 11, while the optical fiber 12 is rotated forward chosing a longitudinal direction of the optical fiber 12 as an axis. On the other hand, when the reversible motor 20 rotates backward, the rod 17 also rotates backward and steps back. As a result, part of the optical fiber 12, which has been wound into the second reel 14, is rewound into the first reel 13, so that the tip portion 12a of the optical fiber 12 rotates reversely and moves in such a direction that it is drawn from the tube 11. The control circuit (not illustrated) for controlling the reversible motor 20 is provided with a timer. The reversible motor 20 switches over repeatedly the forward rotation and the backward rotation whenever the timer counts up, whereby the tip portion 12a of the optical fiber 12 repeats periodical reciprocation and rotational motion within the tube 11.

Incidentally, according to the present embodiment as described above, there is shown an example in which both the reciprocation and the rotational motion are applied to the tip portion 12a of the optical fiber 12. However, it is noted that modifications can be made.

To use the cancer therapeutic instrument 10 shown in Fig. 1, photosensitizer is injected into the affected part, the tube 11 is inserted into the affected part, the tip portion 12a of the optical fiber 12 is inserted into the tube 11, and operation is initiated after a predetermined standby time lapses.

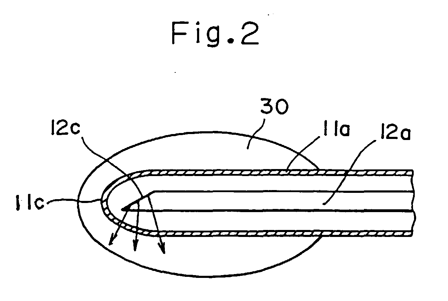

Fig. 2 is a partially enlarged view showing the tip portion 11a of the tube 11 inserted into the affected part 30 and the tip portion 12a of the optical fiber 12 inserted into the tube 11.

As seen from Fig. 2, the tip 11c of the tip portion 11a of the tube 11 is closed so as to prevent body fluid from invading the inside. The tip of the tip portion 12a of the optical fiber 12, which is inserted up to the tip portion 11a of the tube 11, is provided with the tip plane 12c formed by cutting the tip obliquely as shown in the figure. The laser beam travelled through the inside of the optical fiber 12 is reflected by the tip plane 12c and be emitted through the side surface of the tube 12, so that the affected part 30 is irradiated by the laser beam emitted through the side surface of the tube 12.

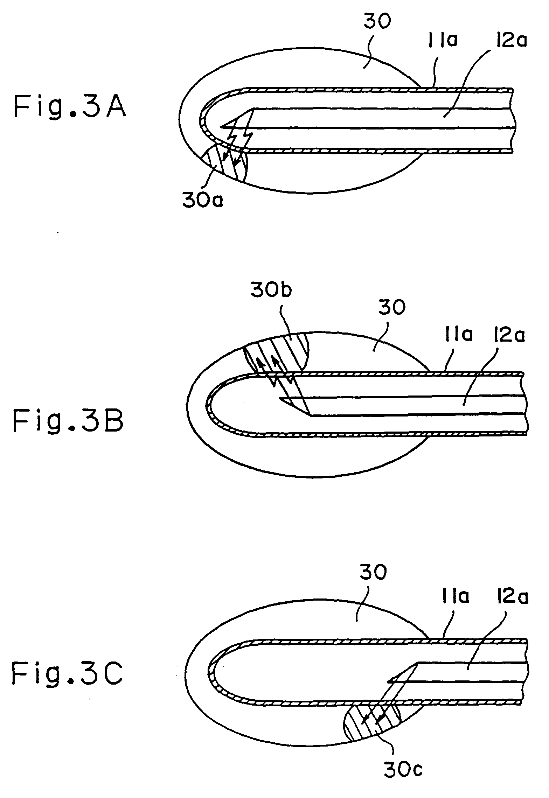

Figs. 3(A)-3(C) are each a view useful for understanding a variation of irradiation area of laser beams according to movement of the optical fiber.

In a state shown in Fig. 3(A), a partial area 30a of the affected part 30 is irradiated with the laser beam. In this state, the tip portion 12a of the optical fiber 12 is rotated and moved in such a direction that it is drawn from the tube 11. At that time, another partial area 30b of the affected part 30 is irradiated with the laser beam as shown in Fig. 3(B). When the tip portion 12a of the optical fiber 12 is rotated and moved to assume the state shown in Fig. 3(A), additional partial area 30c of the affected part 30 is irradiated with the laser beam. In this manner, reciprocation and rotation of the tip portion 12a of the optical fiber 12 inside the tube 11 permits the affected part 30 to be repeatedly irradiated with the laser beams throughout the affected part 30 uniformly.

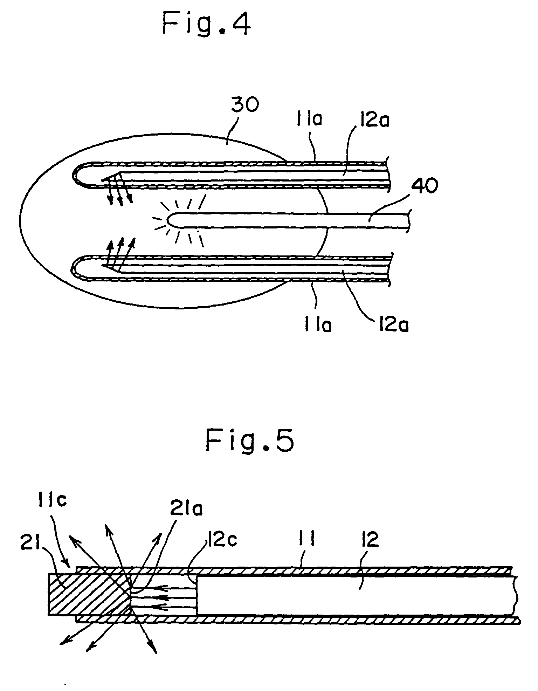

Fig. 4 is a view showing a state in which two tubes 11 are inserted into the affected part 30, and in addition a heat pipe 40 is inserted into the affected part 30.

In the case where the affected part 30 is large, it is acceptable to provide an arrangement in which a plurality of tubes 11 are inserted as shown in the figure, a plurality of optical fibers 12 emit laser beams, and the affected part 30 is irradiated by the laser beams in one treatment. The continuous irradiation with the laser beams heats the affected part 30. If it is desired to lower the temperature of the affected part 30, it is acceptable that the heat pipe 40 is inserted into the affected part 30 as shown in the figure to cool the affected part 30. Alternatively, it is also acceptable to further heat the affected part 30 through the heat pipe 40 so that the above-mentioned hyperthermia is used in combination with the phototherapy.

Fig. 5 is a partially enlarged view showing the tip portion of a tube and the tip portion of an optical fiber which does not constitute an embodiment of the present invention. It however appears to be useful for its understanding.

The tip 11c of the tube 11 is opens at the end. A scatter member 21 is mounted in the opening of the tip 11c of the tube 11 and fixed thereon. The scatter member 21 serves as a stopper with which the opening is closed up. Both the scatter member 21 and the tube 11 are each made of polyacetal. The optical fiber 12 is inserted inside the tube 11. It is preferable that the optical fiber 12 is feasible in insertion into and drawing from the tube 11. However, during the treatment, the optical fiber 12 is disposed at a predetermined position inside the tube 11, and is not moved and rotated.

The tip plane 12c of the optical fiber 12 is cut perpendicularly. The rear end 21a of the scatter member 21 is formed to come face to face with the tip plane 12c of the optical fiber 12, so that part of the laser beams emitted from the tip plane 12c of the optical fiber 12 is reflected on the rear end 21a of the scatter member 21 and then projected from the tube 11, and the other part is projected through the inside of the scatter member 21 from the side of the tube or the tip of the scatter member 21. With such a simple structure, there is realized a non-directional light source which is capable of projecting the laser beams from the tip in all directions. This light source is able to project the laser beams in all directions so that the tube can be inserted into the affected part and the optical fiber can be inserted into the tube and rested. Such a light source is effective particularly in a case where the affected part is small.

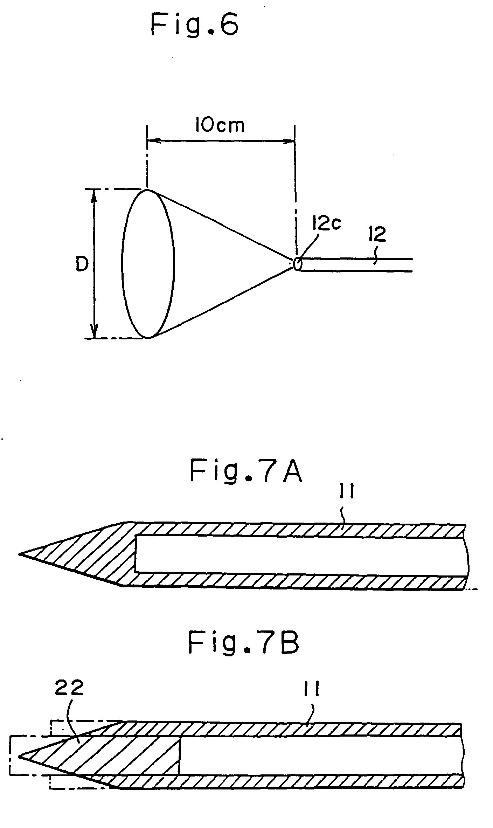

Fig. 6 is a view showing the tip portion of an optical fiber and the state of spread of emitted light according to still another embodiment of a cancer therapeutic instrument of the present invention.

While the tip plane 12c of the optical fiber 12 is cut perpendicularly, the tip plane 12c is subjected to rough grinding by a file. Thus, the laser beam emitted from the tip plane 12c is projected as being spread so that a beam of light at the position advanced by 10 cm from the tip plane 12c is given with its diameter D≧ 2cm.

In this manner, the optical fiber 12, of which the tip plane 12c is processed so that the laser beam is projected as being spread, is inserted into the tube and the affected part is irradiated with the laser beam with the optical fiber 12 rested if the affected part is small, whereas while the optical fiber 12 is reciprocated if the affected part is large.

Figs. 7 (A) and (B) are each an enlarged view showing the tip of a tube.

In Fig. 7 (A), the tip portion of the tube 11 of which the tip is closed is processed in a pin shaped sharp configuration. In Fig. 7 (B), a stopper is mounted in the tip portion of the tube 11 of which the tip is opened, and the tip portion including the stopper is processed in a pin shaped sharp configuration. Incidentally, it is noted that the dashed lines in Fig. 7 (B) show the configuration before process. In this manner, providing sharpness of the tip portion of the tube 11 makes it possible to smoothly insert the tube 11 into the affected part.

Incidentally, there is no need to sharpen the tip portion of the tube 11 in as a cone. It is sufficient for inexpensive processing such as a simple oblique cut, a triangular pyramid - like shaped cut and the like to be adopted.

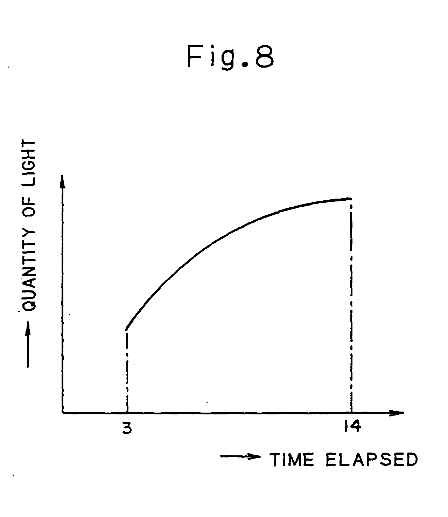

Fig. 8 is a graph which exemplarily shows a variation of an amount of light emitted from a laser light source 15 (cf. Fig.1) according to time elapsed.

In the first stage of an effective period of light therapy shown in Fig. 9, some measure of photosensitizer will still remain also in the normal cells. Thus, light beams emanate with a relatively slight quantity of light at first, and thereafter the quantity of light is increased as the photosensitizer is excreted from the normal cells according to the time elapsed. This control makes it possible to obtain the maximum effect in treatment, limiting harm or damage to normal cells to a minimum.

As described above, according to the present invention, it is possible to obtain a great effect in phototherapy through effective irradiation with activation light on the affected part. In addition it is possible to provide a cancer therapeutic instrument which is capable of reducing the burden on a patient and which is excellent in safety and sanitation since the tube is disposable.

## Claims

1. A cancer therapeutic instrument (10) comprising:
a catheter having a tube (11) whose tip (11a) is closed at the distal end and which is adapted to be inserted into a subject to be treated,
an optical fibre (12) adapted to be slidably disposed inside the tube (11),
a light source (15) emitting activation light which is arranged so as to introduce the activation light into one end of the optical fibre (12) contained within the tube (11) so that the activation light travels within the optical fibre (12) and emanates from the other end of the optical fibre, and
a driving mechanism, characterised in that
the driving mechanism comprises a motor (20), a fixed bearing (18) and a reel (14), wherein the motor (20) and the reel (14) are joined via a rod (17) incorporating a screw thread and being associated with a fixed bearing (18), and wherein the optical fibre (12) is secured to the reel (14) by means of reel fixture (14a) so that operation of the motor (20) serves to displace the reel (14) relative to the fixed bearing (18) and the tube (11) so as to drive that part of the optical fibre (12) between the tip (11a) and reel fixture (14a) in such a manner that the optical fibre (12) within tube (11) is capable of periodic reciprocation in the longitudinal direction of the tube (11) and periodic rotational motion about the longitudinal axis of the tube (11).

2. An instrument (10) as claimed in claim 1, wherein the tip (11a) of the optical fibre is formed as an oblique plane with respect to the longitudinal direction of the optical fibre (12).

3. An instrument (10) as claimed in claim 1 or 2, wherein the tube (11) is provided with a scatter member (21) adapted to scatter activation light.

4. An instrument (10) as claimed in claim 3, wherein the scatter member (21) is made of plastics material containing polyurethane or polyacetal.

5. An instrument (10) as claimed in any preceding claim, wherein the emission solid angle is not less than 0.37 times a maximum and wherein the tip of the optical fibre (12) is formed as a plane having an emission solid angle of not less than 0.12 steradian.

6. An instrument (10) as claimed in any preceding claim, wherein the distal end of the tube (11) is formed to provide a cutting plane whose area in the plane perpendicular to the longitudinal direction of the tube (11) decreases in the direction of the tip of the tube (11).

7. An instrument (10) as claimed in any preceding claim, wherein the light source (15) includes means to continuously or stepwise increase the amount of activation light entering the optical fibre (12) as incident light with the elapse of time.

## Patentansprüche

1. Krebs-Therapieinstrument (10), umfassend:
einen Katheter mit einem Rohr (11), dessen Spitze (11a) am distalen Ende geschlossen ist und welches in einen zu behandelnden Patienten einführbar ist,
eine optische Faser (12), die geeignet ist, innerhalb des Rohres (11) verschiebbar angeordnet zu werden,
eine Aktivierungslicht emittierende Lichtquelle (15), die derart angeordnet ist, dass das Aktivierungslicht in ein Ende der in dem Rohr (11) enthaltenen optischen Faser (12) derart eingeführt wird, dass das Aktivierungslicht sich innerhalb der optischen Faser (12) ausbreitet und an dem anderen Ende der optischen Faser austritt,
und einen Antriebsmechanismus,
**dadurch gekennzeichnet,**
dass der Antriebsmechanismus einen Motor (20), ein festes Lager (18) und eine Spule (14) umfaßt,
wobei der Motor (20) und die Spule (14) über eine Stange (17) verbunden sind, in die ein Schraubgewinde einbezogen ist und die einem festen Lager (18) zugeordnet ist,
und wobei die optische Faser (12) an der Spule (14) mittels eines Spulenhalters (14a) derart befestigt ist, dass der Betrieb des Motors (20) dazu dient, die Spule (14) relativ zu dem festen Lager (18) und dem Rohr (11) derart zu verschieben, dass der betreffende Teil der optischen Faser (12) zwischen der Spitze (11a) und dem Spulenhalter (14a) in einer solchen Weise angetrieben wird, dass die optische Faser (12) innerhalb des Rohres (11) eine periodische Hin- und Herbewegung in der Längsrichtung des Rohres (11) und eine periodische Drehbewegung um die Längsachse des Rohres (11) ausführen kann.

2. Instrument (10) nach Anspruch 1, wobei die Spitze (11a) der optischen Faser als eine schräge Ebene in bezug auf die Längsrichtung der optischen Faser (12) ausgebildet ist.

3. Instrument (10) nach Anspruch 1 oder 2, wobei das Rohr (11) mit einem Streuteil (21) versehen ist, welches Aktivierungslicht zu streuen imstande ist.

4. Instrument (10) nach Anspruch 3, wobei das Streuteil (21) aus Polyurethan oder Polyacetal enthaltendem Kunststoffmaterial hergestellt ist.

5. Instrument (10) nach irgendeinem vorhergehenden Anspruch, wobei der Emissions-Raumwinkel nicht kleiner als das 0,37-fache eines Maximums ist und wobei die Spitze der optischen Faser (12) als eine Ebene ausgebildet ist, die einen Emissions-Raumwinkel von nicht weniger als 0,12 Steradiant aufweist.

6. Instrument (10) nach irgendeinem vorhergehenden Anspruch, wobei das distale Ende des Rohres (11) derart ausgebildet ist, dass eine Schnittebene geschaffen ist, deren Fläche in der rechtwinklig zur Längsrichtung des Rohres (11) verlaufenden Ebene in Richtung der Spitze des Rohres (11) abnimmt.

7. Instrument (10) nach irgendeinem vorhergehenden Anspruch, wobei die Lichtquelle (15) eine Einrichtung enthält, welche die Menge des in die optische Faser (12) als einfallendes Licht eintretenden Aktivierungslichts im Laufe der Zeit kontinuierlich oder schrittweise steigert.

## Revendications

1. Instrument de thérapie du cancer (10) comportant :
- un cathéter ayant un tube (11) dont la pointe (11a) est fermée à l'extrémité distale et qui est adapté pour être inséré dans un sujet à traiter,
- une fibre optique (12) adaptée pour être disposée de façon coulissante à l'intérieur du tube (11),
- une source de lumière (15) émettant une lumière d'activation, disposée de façon à introduire la lumière d'activation dans une extrémité de la fibre optique (12) contenue dans le tube (11) de sorte que la lumière d'activation chemine à l'intérieur de la fibre optique (12) et émane à l'autre extrémité de cette fibre optique, et,
- un mécanisme d'entraînement, caractérisé en ce que le mécanisme d'entraînement comporte un moteur (20), un palier fixe (18) et une bobine (14), en ce que le moteur (20) et la bobine (14) sont reliés par l'intermédiaire d'une tige (17) munie d'un filetage et associée au palier fixe (18), et en ce que la fibre optique (12) est fixée à la bobine (14) au moyen d'une fixation de bobine (14a) de sorte que le fonctionnement du moteur (20) serve à déplacer la bobine (14) par rapport au palier fixe (18) et au tube (11) de façon à entraîner la partie de la fibre optique (12) entre la pointe (11a) et la fixation de bobine (14a) de telle manière que la fibre optique (12) à l'intérieur du tube (11) puisse être animée d'un mouvement alternatif périodique dans la direction longitudinale du tube (11) et d'un mouvement de rotation périodique autour de l'axe longitudinal du tube (11).

2. Instrument (10) suivant la revendication 1, caractérisé en ce que la pointe (11a) de la fibre optique forme un plan oblique par rapport à la direction longitudinale de cette fibre optique (12).

3. Instrument (10) suivant la revendication 1 ou 2, caractérisé en ce que le tube (11) est muni d'un organe de dispersion (21) adapté pour disperser la lumière d'activation.

4. Instrument (10) suivant la revendication 3, caractérisé en ce que l'organe de dispersion (21) est fait d'un matériau plastique contenant cm polyuréthane ou du polyacétal.

5. Instrument (10) suivant une quelconque des revendications précédentes, caractérisé en ce que l'angle solide d'émission n'est pas inférieur à 0,37 fois un maximum et en ce que la pointe de la fibre optique (12) forme un plan ayant un angle solide d'émission non inférieur à 0,12 stéradian.

6. Instrument (10) suivant une quelconque des revendications précédentes, caractérisé en ce que l'extrémité distale du tube (11) est formée pour fournir un plan de découpe dont la surface dans le plan perpendiculaire à la direction longitudinale du tube (11) diminue dans la direction de la pointe du tube (11).

7. Instrument (10) suivant une quelconque des revendications précédentes, caractérisé en ce que la source de lumière (15) comporte des moyens pour augmenter continuellement ou par degré la quantité de lumière d'activation pénétrant dans la fibre optique (12) en tant que lumière incidente, avec le temps écoulé.
